# EUROPEAN PATENT APPLICATION

(11) **EP 1 970 099 A2**
(43) Date of publication of application: **17.09.2008**
(21) Application number: 08300129.7
(22) Date of filing: 03.03.2008
(51) Int. Cl.: A61Q 5/04, A61K 8/41, A61K 8/49, A61K 8/24, A61K 8/19, A61K 8/36, A61K 8/365

(54) **Process for relaxing or straightening hair**

(30) Priority: 14.03.2007 US 717824
(71) Applicant: L'Oreal, 75008 Paris (FR)
(72) Inventor: Cannel, David W., Plainfiled, NJ 07060 (US); Chong Espino, Cynthia, Princeton, NJ 08540 (US); Van Nguyen, Nghi, Edison, NJ 08820 (US); Genain, Gilles, 75116, Paris (FR)
(74) Representative: Martin-Charbonneau, Virginie

(57) **Abstract**

The present invention relates to a process for straightening or relaxing hair comprising:
(a) pre-alkalizing the hair by contacting it with an alkaline composition having a pH of from 8.0 to 10.5 to form pre-alkalized hair;
(b) applying onto the pre-alkalized hair a hair straightening/relaxing composition containing:
(i) from 0.1% to 50% by weight of at least one weak non-hydroxide base; and
(ii) remainder, to 100%, a cosmetically acceptable medium;
to form treated hair, and
(c) smoothing the hair using a combination of heat and means for physically smoothing hair; the smoothing step (c) being performed prior to and/or after the application step (b) of the hair straightening/relaxing composition.

## Description

Hair straightening or hair relaxing products have been commercially available for over fifty years for people who want straighter, more manageable hair. Most commercially available hair relaxers are composed of a strong hydroxide base that breaks the bonds in the hair.

Commercial products based only on alkaline metal hydroxides such as sodium hydroxide and lithium hydroxide are typically used to straighten or relax curly/kinky hair. There are primarily four different types of alkaline metal hydroxide hair straighteners in use: calcium hydroxide, lithium hydroxide, sodium hydroxide, and potassium hydroxide. The straightening product is usually applied quickly and can only remain in the hair for a very limited amount of time. Due to the alkalinity of such products, if the product is not rinsed from the hair at the appropriate time, damage to the hair can occur, as well as chemical burns to the scalp and areas surrounding the hair.

Thus, the object of the present invention is to provide a hair straightening or relaxing process which is safer than, yet as effective as, conventional processes.

The present invention is directed to a process for straightening or relaxing hair involving the steps of:

(a) pre-alkalizing the hair by contacting it with an alkaline composition having a pH of from 8.0 to 10.5 to form pre-alkalized hair;

(b) applying onto the pre-alkalized hair a hair straightening/relaxing composition containing:
(i) from 0.1% to 50% by weight of at least one weak non-hydroxide base; and
(ii) remainder, to 100%, a cosmetically acceptable medium; and
(c) smoothing the hair using a combination of heat and means for physically smoothing the hair, the smoothing step (c) being performed prior to and/or after the application step (b) of the hair straightening/relaxing composition.

Other than in the operating examples, or where otherwise indicated, all numbers expressing quantities of ingredients and/or reaction conditions, are to be understood as being modified in all instances by the term "about".

It has been surprisingly found that by employing the process of the present invention, straightening/relaxing of hair can be achieved in a manner which is less harmful to a user's skin and hair than conventional hair straightening/relaxing processes.

Conventional products, which employ large amounts of hydroxide, have a tendency to cause skin irritation, as well as damage to the hair itself, due to the use of large amounts of hydroxide in said products. However, by first pre-alkalizing the hair, followed by treating it with a weak non-hydroxide base product, and smoothing the hair by employing a combination of heat and means for physically smoothing the hair, satisfactory straightening/relaxing of the hair can be achieved in a manner that is less harmful to both skin and hair.

The pre-alkalizing step of the present invention involves the use of an alkaline composition having a pH ranging from 8.0 to 10.5, preferably from 8.5 to 9.5. Any conventional base, whether alkaline hydroxide or non-hydroxide, may be employed so long as it results in the formation of an alkaline composition having the above-disclosed pH range. The precise amount of conventional base used will depend on the specific base(s) chosen. Once the hair has been pre-alkalized, the alkaline composition may, optionally, be rinsed-off before performing the next step of the process of the present invention.

The purpose of the pre-alkalizing step is to open the hair cuticle, thereby rendering it more susceptible to the subsequent penetration of the non-hydroxide base. This in turn renders the hair straightening/relaxing process more efficient and less time-consuming.

The alkaline composition may be employed in any suitable form. Examples thereof include, but are not limited to, an alkaline shampoo, an alkaline conditioner or an alkaline solution in general. In a particularly preferred embodiment, the alkaline composition is in the form of an alkaline shampoo which would facilitate both the pre-alkalizing and cleaning of the hair at the same time.

Suitable weak non-hydroxide bases for use in the present invention are preferably those bases having a pKa of from 2 to 13, preferably from 3 to 11.

The weak non-hydroxide bases may be chosen from weak organic bases and weak inorganic bases.

Weak organic bases useful in the present invention include nitrogen-containing bases, which do not completely disassociate in water. Examples thereof include, but are not limited to, amines such as ethylamines, ethyleneamines, ethanolamines, including cyclic amines such as for example aniline, quinoline and other cyclic compounds, saturated or unsaturated, having one or more nitrogen atoms within the ring.

Examples of five-membered rings having one nitrogen atom include, but are not limited to, pyrroline, pyrrole, pyrrolidine, and derivatives thereof.

Examples of five-membered rings having two nitrogen atoms include, but are not limited to, pyrazole, pyrazoline, imidazolidine, imidazole, imidazoline, and derivatives thereof.

Examples of six-membered rings having one nitrogen atom include, but are not limited to, morpholine, pyridine, piperidine, and derivatives thereof.

Examples of six-membered rings having two nitrogen atoms include, but are not limited to, pyridazine, pyrimidine, pyrazine, piperazine, and derivates thereof.

Examples of six-membered rings having three nitrogen atoms include, but are not limited to, triazine, and derivatives thereof.

Particularly preferred weak organic bases include ethylenediamines, monoethanolamines, imidazole, pyrrole, pyrrolidine, and mixtures thereof.

Also preferred are mixtures of the above mentioned weak organic bases, such as a mixture of ethylenediamine and imidazole, or a mixture of monoethanolamine and imidazole.

Weak inorganic bases useful in the present invention include alkali metal phosphates and carbonates such as, for example, sodium phosphate, potassium phosphate, sodium carbonate, sodium bicarbonate, potassium carbonate, potassium bicarbonate, and their derivatives.

Weak inorganic bases may also include alkali metals of carboxylates such as, for example, sodium acetate, potassium acetate, sodium citrate, and potassium citrate, and their derivatives.

Particularly preferred weak inorganic bases include potassium phosphate, sodium phosphate, and sodium carbonate.

The weak non-hydroxide base is employed in the hair straightening/relaxing composition in an amount of from 0.1% to 50% by weight, preferably from 0.1% to 30% by weight, more preferably from 0.1% to 10% by weight, based on the total weight of the composition.

As used herein, the term "cosmetically acceptable medium" is known to one of ordinary skill in the art, and may comprise, for example, water and/or at least one organic solvent. It may further comprise one or more adjuvants.

The hair straightening/relaxing composition disclosed herein may be, for example, in the form of a thickened cream so as to hold the hair as stiff as possible. These creams are made in the form of "heavy" emulsions, for example, based on glyceryl stearate, glycol stearate, self-emulsifying waxes, fatty alcohols, mineral oil and petrolatum.

Liquids or gels containing thickeners, such as carboxyvinyl polymers or copolymers that "stick" the hair together and hold them in a smooth position during the leave-in time, may also be used.

The cosmetically acceptable medium may comprise at least one adjuvant chosen, for example, from silicones in soluble, dispersed and microdispersed forms, nonionic, anionic, cationic and amphoteric surfactants, ceramides, glycoceramides and pseudoceramides, vitamins and provitamins including panthenol, waxes other than ceramides, glycoceramides and pseudoceramides, water-soluble and liposoluble, silicone-based and non-silicone-based sunscreens, nacreous agents and opacifiers, sequestering agents, plasticizers, solubilizers, acidifying agents, mineral and organic thickeners, antioxidants, hydroxy acids, penetrating agents, fragrances, and preserving agents.

In the event that surfactants are employed, said hair straightening/relaxing composition may be used as a shampoo. Similarly, in the event that one were to decide to use said hair straightening/relaxing composition as a hair conditioner, various types of conditioning agents can be added to the composition in order to facilitate this hair treating property.

Smoothing of hair according to the present invention involves using a combination of heat and means for physically smoothing the hair. The heat necessary to effectuate smoothing should be at least 50°C; preferably at least 75°C; more preferably at least 100°C. The precise amount of heat employed will depend on the concentration of the weak non-hydroxide base present in the composition. This heat may emanate from any suitable source such as, for example, a hair dryer or hot/flat iron.

The means for physically smoothing hair can be any apparatus capable of physically smoothing the hair such as, for example, a hair brush or comb. In one embodiment, the means for smoothing hair also serves as the source for generating heat such as, for example, a hot/flat iron.

According the present invention, the pre-alkalized hair may be contacted with the above-disclosed hair straightening/relaxing composition for a period of less than 60 minutes, preferably less than 40 minutes, more preferably less than 30 minutes, even more preferably less than 20 minutes. According to a preferred embodiment of the present invention, the pre-alkalized hair is contacted with the above-disclosed hair straightening/relaxing composition at a temperature of less than 30°C, preferably at room temperature (about 25°C). Then, the treated hair may optionally be rinsed.

Optionally, the pre-alkalized hair or the treated hair may further be contacted with a non-volatile oil chosen from plant, animal, mineral and synthetic oils, and mixtures thereof.

As is disclosed above, the hair straightening/relaxing composition may either be left on the hair, or rinsed out. As for the non-volatile oil, if employed, it will preferably remain on the hair.

According to one preferred embodiment of the present invention, there is provided a process for straightening or relaxing hair involving the steps of:
(a) pre-alkalizing the hair by contacting it with an alkaline composition having a pH of from about 8.0 to about 10.5 to form pre-alkalized hair;
(b) optionally, rinsing the alkaline composition from the pre-alkalized hair;
(c) providing a hair straightening/relaxing composition containing:
   (i) from about 0.1 to about 50% by weight of at least one weak non-hydroxide base; and
   (ii) remainder, to 100%, a cosmetically acceptable medium, all weights based on the weight of the hair straightening/relaxing composition;
(d) contacting the hair with the hair straightening/relaxing composition to form treated hair;
(e) optionally, rinsing the hair straightening/relaxing composition from the treated hair;
(f) optionally, contacting the treated hair with a non-volatile oil; and
(g) smoothing the hair using a combination of heat and means for physically smoothing hair to form smoothed hair.

According to another preferred embodiment of the present invention, there is provided a process for straightening or relaxing hair involving the steps of:
(a) pre-alkalizing the hair by contacting it with an alkaline composition having a pH of from 8.0 to 10.5 to form pre-alkalized hair;
(b) optionally, contacting the pre-alkalized hair with a non-volatile oil chosen from plant, animal, mineral and synthetic oils;
(c) smoothing the hair using a combination of heat and means for physically smoothing hair to form smoothed hair;
(d) contacting the smoothed hair with the hair straightening/relaxing composition to form treated hair;
(e) optionally, rinsing the hair straightening/relaxing composition from the treated hair after it has been in contact with the hair for a period of less than 60 minutes; and
(f) optionally, smoothing the treated hair using a combination of heat and means for physically smoothing hair to form smoothed hair.

It should be noted that the steps of: contacting the hair with a non-volatile oil; and smoothing the hair, may, independently from each other, be performed prior to and/or after application of the hair straightening/relaxing composition.

In commercially available hair straightening or relaxing compositions, a highly caustic hydroxide compound such as sodium hydroxide must be used in order to satisfactorily straighten/relax the hair without heat. In the present invention, however, a less caustic and less concentrated non-hydroxide compound can be used because of the synergy realized by using a combination of heat and an apparatus capable of physically smoothing the hair. Without intending to be bound by theory, it is believed that a synergistic effect in hair straightening/relaxing is realized due to an induced supercontraction and denaturation of hair protein caused by the combination of heat and physical smoothing.

Moreover, due to the use of a less caustic and less concentrated non-hydroxide compound, a barrier substance is not required when using the hair straightening/relaxing composition of the present invention. Commercially available hair relaxing products oftentimes require the hair stylist to apply a barrier substance such as petrolatum to the skin surrounding the scalp and the area around the ears. The barrier substance is used to prevent the skin from becoming irritated if the hair relaxing product contacts the skin. A barrier substance is not necessary when using the process of the present invention because the concentration and the irritation of the non-hydroxide compound is much lower.

The present invention will be better understood from the examples which follow, all of which are intended for illustrative purposes only, and are not meant to unduly limit the scope of the invention in any way.

### EXAMPLES

The Percentage Straightening Efficiency (%SE) was calculated using the following formula:

% SE = (A/B) × 100, where A = final length measured (cm), B = initial length of hair = 10cm (full length of hair when straight).

The following examples show the % SE of hair treated with various protocols.

### Example 1.

In this example, kinky hair was pre-treated with either a conventional acid shampoo (15% by weight sodium lauryl ether sulfate, pH 4.34) or an alkaline shampoo (15% by weight sodium lauryl ether sulfate, pH 9.65) for 2 minutes, rinsed for 10 seconds, blot-dried, then soaked in a 2% by weight MEA (monoethanolamine) solution in water (10 minutes at room temperature (RT) or 10 minutes at 50°C), blot-dried, then straightened with a flat iron at 400°F, ie 204,4°C (3 passes/6 seconds each). The hair was then shampooed and the % SE was determined as described above.
The results obtained are the following:

| | Control (No Treatment) | Acid Shampoo, 2% MEA at RT | Acid Shampoo, 2% MEA at 50° | Alkaline Shampoo, 2% MEA at RT |
|---|---|---|---|---|
| %SE | 46% | 76% | 83% | 82% |

The results showed that pre-alkalizing the hair and treating the hair with MEA at room temperature will improve the % SE to the same extent as treating the hair with MEA at high temperature.

### Example 2.

Following the same protocol as described in Example 1, kinky hair was pre-alkalized using the alkaline shampoo then treated with various solutions of pyrrolidine in water at room temperature. The %SE are shown below.

| | 0.1% by weight pyrrolidine | 0.5% by weight pyrrolidine | 1% by weight pyrrolidine | 4.1% by weight pyrrolidine |
|---|---|---|---|---|
| %SE | 55% | 77% | 94% | 97.5% |

The results showed that as the concentration of the cyclic amine increased, the %SE increased as well.

## Claims

1. A process for straightening or relaxing hair comprising:
(a) pre-alkalizing the hair by contacting it with an alkaline composition having a pH of from 8.0 to 10.5 to form pre-alkalized hair;
(b) applying onto the pre-alkalized hair a hair straightening/relaxing composition containing:
(i) from 0.1% to 50% by weight of at least one weak non-hydroxide base; and
(ii) remainder, to 100%, a cosmetically acceptable medium;
to form treated hair, and
(c) smoothing the hair using a combination of heat and means for physically smoothing hair;
the smoothing step (c) being performed prior to and/or after the application step (b) of the hair straightening/relaxing composition.

2. The process of claim 1 wherein after step (a), the alkaline composition is rinsed-off before performing the next step.

3. The process of any preceding claim, wherein the at least one weak non-hydroxide base is employed in an amount of from 0.1 to 30% by weight, based on the total weight of the hair straightening/relaxing composition.

4. The process of the preceding claim, wherein the at least one weak non-hydroxide base is employed in an amount of from 0.2 to 15% by weight, based on the total weight of the hair straightening/relaxing composition.

5. The process of any preceding claim, wherein the at least one weak non-hydroxide base has a pKa of from 2 to 13.

6. The process of any preceding claim, wherein the at least one weak non-hydroxide base is a nitrogen-containing base.

7. The process of the preceding claim, wherein the at least one weak non-hydroxide base is chosen from amines such as ethylamines, ethyleneamines, ethanolamines, including cyclic amines such as aniline, quinoline and other cyclic compounds, saturated or unsaturated, having one or more nitrogen atoms within the ring.

8. The process of claim 6, wherein the at least one weak non-hydroxide base is chosen from :
- pyrroline, pyrrole, pyrrolidine, and derivatives thereof;
- pyrazole, pyrazoline, imidazolidine, imidazole, imidazoline, and derivatives thereof;
- morpholine, pyridine, piperidine, and derivatives thereof;
- pyridazine, pyrimidine, pyrazine, piperazine, and derivates thereof;
- triazine, and derivatives thereof;
- and mixtures of the above mentioned compounds.

9. The process of claim 6, wherein the at least one weak non-hydroxide base is chosen from ethylenediamines, monoethanolamines, imidazole, pyrrole, pyrrolidine, and mixtures thereof.

10. The process of claim 6, wherein the at least one weak non-hydroxide base is a mixture of ethylenediamine and imidazole.

11. The process of claim 6, wherein the at least one weak non-hydroxide base is a mixture of monoethanolamine and imidazole.

12. The process of anyone of claims 1 to 5, wherein the at least one weak non-hydroxide base is chosen from alkali metal phosphates, alkali metal carbonates and alkali metals of carboxylates, and mixtures thereof.

13. The process of claim 12, wherein the at least one weak non-hydroxide base is chosen from:
- sodium phosphate, potassium phosphate, sodium carbonate, sodium bicarbonate, potassium carbonate, potassium bicarbonate, and their derivatives;
- sodium acetate, potassium acetate, sodium citrate, and potassium citrate, and their derivatives;
- and mixtures thereof.

14. The process of any preceding claim, wherein the pre-alkalized hair is contacted with the straightening/relaxing composition for a period of less than 60 minutes.

15. The process of the preceding claim, wherein the pre-alkalized hair is contacted with the straightening/relaxing composition for a period of less than 40 minutes

16. The process of any preceding claim, wherein after the pre-alkalized hair has been contacted with the straightening/relaxing composition, the treated hair is rinsed.

17. The process of any preceding claim, wherein the pre-alkalized hair or the treated hair is further contacted with a non-volatile oil chosen from plant, animal, mineral and synthetic oils, and mixtures thereof.

18. The process of any preceding claim, wherein the heat employed in the smoothing step is at least 50°C.

19. The process of the preceding claim, wherein the heat employed in the smoothing step is at least 100°C.

20. The process of any preceding claim, wherein the process is performed without the use of a barrier substance.

21. The process of any preceding claim, wherein the means for physically smoothing hair is chosen from a brush and a comb.

22. The process of any preceding claim, wherein the smoothing step is performed using a hot/flat iron at a temperature of at least 100°C.

23. A process for straightening or relaxing hair involving the steps of:
(a) pre-alkalizing the hair by contacting it with an alkaline composition having a pH of from about 8.0 to about 10.5 to form pre-alkalized hair;
(b) optionally, rinsing the alkaline composition from the pre-alkalized hair;
(c) providing a hair straightening/relaxing composition containing:
(i) from about 0.1 to about 50% by weight of at least one weak non-hydroxide base; and
(ii) remainder, to 100%, a cosmetically acceptable medium, all weights based on the total weight of the hair straightening/relaxing composition;
(d) contacting the hair with the hair straightening/relaxing composition to form treated hair;
(e) optionally, rinsing the hair straightening/relaxing composition from the treated hair;
(f) optionally, contacting the treated hair with a non-volatile oil; and
(g) smoothing the hair using a combination of heat and means for physically smoothing hair to form smoothed hair.

24. A process for straightening or relaxing hair involving the steps of:
(a) pre-alkalizing the hair by contacting it with an alkaline composition having a pH of from 8.0 to 10.5 to form pre-alkalized hair;
(b) optionally, contacting the pre-alkalized hair with a non-volatile oil chosen from plant, animal, mineral and synthetic oils;
(c) smoothing the hair using a combination of heat and means for physically smoothing hair to form smoothed hair;
(d) contacting the smoothed hair with a hair straightening/relaxing composition containing:
(i) from about 0.1 to about 50% by weight of at least one weak non-hydroxide base; and
(ii) remainder, to 100%, a cosmetically acceptable medium, all weights based on the total weight of the hair straightening/relaxing composition,
to form treated hair;
(e) optionally, rinsing the hair straightening/relaxing composition from the treated hair after it has been in contact with the hair for a period of less than 60 minutes; and
(f) optionally, smoothing the treated hair using a combination of heat and means for physically smoothing hair to form smoothed hair.
